# EUROPEAN PATENT SPECIFICATION

(11) **EP 1 534 252 B1**
(45) Date of publication and mention of the grant of the patent: **30.06.2010**
(21) Application number: 03791762.2
(22) Date of filing: 26.08.2003
(51) Int. Cl.: A23G 3/00, A61K 8/92, A61Q 11/00, A61K 9/00

(54) **BREATH FRESHENING AND ORAL CLEANSING PRODUCT USING CARDAMOM OIL**
ATEMERFRISCHER UND MUNDPFLEGEPRODUKT UNTER VERWENDUNG VON KARDAMOM L
PRODUIT PURIFIANT L'HALEINE ET PRODUIT PURIFICATEUR BUCCAL A L'ESSENCE DE CARDAMOME

(30) Priority: 27.08.2002 US 319498 P
(43) Date of publication of application: 01.06.2005
(73) Proprietor: WM. WRIGLEY JR. COMPANY, Chicago, Illinois 60611 (US)
(72) Inventor: MAXWELL, James, Chicago, IL 60640 (US); GREENBERG, Michael, Northbrook, IL 60062 (US); STAWSKI, Barbara, Forest Park, IL 60130 (US); BRODERICK, Kevin, Berwyn, IL 60402 (US)
(74) Representative: Hayes, Adrian Chetwynd
(86) International application number: PCT/US2003/026617
(87) International publication number: WO 2004/019867

(56) References cited:
- WO-A-02/43657
- US-A- 4 312 766
- US-A- 4 351 347
- US-A- 4 626 427
- US-A- 4 775 537
- US-A- 4 820 544
- US-A- 4 820 544
- US-A1- 2001 022 964
- US-A1- 2001 022 964
- US-B1- 6 365 215
- US-B1- 6 365 215

## Description

### Background of Invention

There is considerable consumer demand for products that freshen breath and kill bacteria in the mouth. An oral product with breath freshening and bactericidal benefits is a convenient delivery for oral cleansing in the oral cavity and freshening breath.

Of course, breath freshening is a very important part of everyday life. In order to facilitate proper oral hygiene, oral cleansing and breath freshening practices should be conducted repeatedly throughout the day.

However, oral cleansing and breath freshening may be difficult or inconvenient at times, depending on the nature of the breath freshening desired and the situation in which the breath freshening must occur. Brushing, flossing, cleaning your tongue and gargling using a variety of devices and compositions are common oral care practices well-suited for the privacy of one's home. But, such devices and compositions are less convenient to use away from the home where bathroom facilities might be scarce, unavailable or unsanitary.

It is known to incorporate active agents into oral products for the purpose of providing oral benefits including breath freshening and bactericidal properties. Such systems have the advantage of providing a rapid effect and convenient delivery.

### Summary of Invention

The present invention relates to methods of oral cleansing. Furthermore, the present invention relates to the composition of, an oral product. Specifically, the present invention relates to oral products intended for bactericidal and breath freshening properties. More specifically, the present invention relates to a chewing gum or confection containing an .. effective amount of cardamom oil which provides bactericidal properties for oral cleansing and breath freshening, by which the inventive composition effectively inactivates or kills oral bacteria and freshens breath through the consumption of the chewing gum or confection.

In an embodiment of the present invention, the oral product is chewing gum or any variation, including but not limited to, bubble gums, pellets, gum balls or sticks. Chewing gums may be coated or not coated and be of a variety of flavors, shapes and sizes.

In an embodiment of this invention, the oral product is a confectionery composition including but not limited to hard candy, chewing candy, filled candy and pressed tablets.

### Detailed Description

It is known to use chewing gum and confections as a vehicle for delivering components to the oral cavity which provide oral benefits such as breath freshening and bactericidal properties. Such systems have the advantage of providing a consumer with a convenient and inexpensive method for maintaining oral health and fresh breath throughout the course of the day.

The present invention incorporates cardamom oil as the active component for breath freshening and oral bactericidal benefits. Cardamom oil is known to have bactericidal and anti-fungal properties. The cardamom oil used in the present invention was FCC grade and purchased from Sigma-Aldrich Corporation, St. Louis, MO, USA.

In vitro tests were conducted with a gram positive supragingival plaque bacterium, Streptococcus mutans. In addition, two subgingival plaque bacteria associated with oral malodor were tested, Porphyromonas gingivalis and Fusobacterium nucleatum. The MIC (Minimum Inhibitory Concentrations) study protocol is as follows. Chlorhexidine was used as a positive control and sterile water was used as a negative control. Cardamom oil was suspended in 10% methanol with 3.8% Tween 80. No noticeable growth inhibitory effect was seen in the solvent control. 96 well microtiter plates were used for this study. Each well contained 5 x 10⁵ colony forming units/ml of bacteria, serially diluted agents and bacterial growth medium. All bacterial cultures were incubated at 37°C and stationary. Bacterial growth was estimated spectrophotometrically at 660 nm, after 48 hours. The MIC for each test bacteria was defined as the minimum concentration of test compound limiting turbidity to <absorbance at 660nm.

The MBC (Minimum bactericidal concentrations) were determined using the 96 well microtiter plate serial dilutions as described above for MIC studies. Serial dilution of cultures in wells showing no visible growth were performed and 10microliters of culture were plated in triplicate on blood agar plates. Viable colonies were scored after incubation of the plates for 48 hours at 37°C. For each test bacterium, the number of CFU/ml were determined in the initial inoculum. The MBC was defined as the lowest concentration of a test compound that killed at least 99.9% of the cells present in the initial inoculum.

The results of the studies performed to obtain minimum inhibitory concentration (MIC) and minimum bactericidal concentration (MBC) of cardamom oil are as follows. For P. gingivalis, the cardamom oil had an MIC of 250 µg/ml and an MBC of 250µg/ml. For F, nucleatum the cardamom oil had an MIC of 250µg/ml and an MBC of 250µg/ml. For S. mutans the cardamom oil had an MIC of 2000µg/ml and was non-inhibitory against S. mutans. Cholorhexidine was the positive control and produced an MIC and MBC of 1.25µg/ml for both bacteria. The solvent of 10% methanol, 3.8% Tween 80 had no noticeable growth inhibitory effects on either of the bacteria in the study.

In an embodiment, the invention comprises the use of the oral composition comprising cardamom oil in an amount sufficient to kill or deactivate oral bacteria and causing a person in need of the treatment to consume the oral composition whereby the bacteria in the oral cavity of the person is reduced or inactivated by the treatment.

In a preferred embodiment, the oral composition comprises additional breath freshening or oral health ingredients.

In a preferred embodiment, the additional breath freshening or oral health ingredients comprise anti-microbial ingredients.

In a preferred embodiment, the additional breath freshening or oral health ingredients comprise food acceptable salts of zinc or copper.

In a preferred embodiment, the additional breath freshening or oral health ingredients comprise cooling agents. :

In a preferred embodiment, the additional breath freshening or oral health ingredients comprise pyrophosphate or polyphosphate.

In a preferred embodiment, the oral composition is formulated to deliver at least 0.005% concentration of cardamom oil to the oral cavity.

In a preferred embodiment, the oral composition is formulated to deliver at least 0.01 % concentration of cardamom oil to the oral cavity.

In a preferred embodiment, the oral compositions is formulated to deliver at least 0.1% concentration of cardamom oil to the oral cavity.

There are several methods which may be used to enhance the release of the cardamom oil from the oral composition. In a chewing gum product, the gum base is hydrophilic which would facilitate the release of the cardamom oil. In a chewing gum composition, the cardamom oil may be encapsulated, spray dried, formulated into the coating and combinations thereof.

In an embodiment of the present invention, an effective amount for anti-microbial benefit of cardamom oil is present in a chewing gum formulation. The cardamom oil is present in an amount of from 0.01% to 5% by weight of the chewing gum product. In a preferred embodiment of the present invention, the amount of cardamom oil is about 1% of the weight of the chewing gum product. In another preferred embodiment, the cardamom oil is present in the amount of about 0.25% by weight of the chewing gum product. In another preferred embodiment, the cardamom oil is present in the amount of about 0.01 % by weight of the chewing gum product.

In general, a chewing gum composition typically comprises a water soluble portion, a water insoluble gum base portion and typically flavoring agents. The water soluble portion dissipates with a portion of the flavoring agent over a period of time during chewing. The gum base portion is retained in the mouth throughout the chew.

The insoluble gum base generally comprises elastomers, resins, fats and oils, softeners and inorganic fillers. The gum base may or may not include wax. The insoluble gum base can constitute 5% to 95% by weight of the chewing gum, more commonly the gum base comprises 10% to 50% of the gum, and in some preferred embodiments 25% to 35% by weight, of the chewing gum.

In a particular embodiment, the chewing gum base of the present invention contains 20% to 60% by weight synthetic elastomer, up to 30% by weight natural elastomer, 5% to 55% by weight elastomer plasticizer, 4% to 35% by weight filter, 5% to 35% by weight softener, and optional minor amounts (1% or less by weight) of miscellaneous ingredients such as colorants, antioxidants, etc.

Synthetic elastomers may include, but are not limited to, polyisobutylene with GPC weight average molecular weight of 10,000 to 95,000, isobutylene-isoprene copolymer (butyl elastomer), styrenecopolymers having styrene-butadiene ratios of 1:3 to 3:1, polyvinyl acetate having GPC weight average molecular weight of 2,000 to 90,000, polyisoprene, polyethylene, vinyl acetate vinyl laurate copolymer having vinyl laurate content of 5% to 50% by weight of the copolymer, and combinations thereof.

Preferred ranges for polyisobutylene are 50,000 to 80,000 GPC weight average molecular weight and for styrene are 1:1 to 1:3 bound styrene for polyvinyl acetate are 10,000 to 65,000 GBC weight average molecular weight with the higher molecular weight polyvinyl acetates typically used in bubble gum base, and for vinyl acetatelaurate, vinyl laurate content of 10

Natural elastomers may include natural rubber such as smoked or liquid latex and guayule as well as natural gums such as jelutong, lechi caspi, perillo, sorva, massaranduba balata, massaranduba chocolate, nispero, rosindinha, chicle, gutta hang kang, and combinations thereof. The preferred synthetic elastomer and natural elastomer concentrations vary depending on whether the chewing gum in which the base is used is adhesive or conventional, bubble gum or regular gum, as discussed below. Preferred natural elastomers include jelutong, chicle, sorva and massaranduba balata.

Elastomer plasticizers may include, but are not limited to, natural rosin esters such as glycerol esters or partially hydrogenated rosin, glycerol esters of polymerized rosin, glycerol esters of partially dimerized rosin, glycerol esters of rosin, pentaerythritol esters of partially hydrogenated rosin, methyl and partially hydrogenated methyl esters of rosin, pentaerythritol esters of rosin; synthetics - such as terpene resins derived from alpha beta and/or any suitable combinations of the foregoing. The preferred elastomer plasticizers will also vary depending on the specific applicator, and on the type of elastomer which is used.

Filters/texturizers may include magnesium and calcium carbonate, ground limestone, silicate types such as magnesium and aluminum silicate, day, alumina, talc, titanium oxide, mono-, di- and tri-phosphate, cellulose polymers, such as wood, and combinations thereof.

Softeners/emulsifiers may include tallow, hydrogenated tallow, hydrogenated and partially hydrogenated vegetable oils, cocoa butter, glycerol monostearate, glycerol triacetate, lecithin, mono and triglycerides, acetylated monoglycerides, fatty acids (e.g. stearic, palmitic, oleic and linoleic acids), and combinations thereof

Colorants and whiteners may include FD&C dyes and lakes, fruit and vegetable extracts, titanium dioxide, and combinations thereof.

The base may or may not include wax. An example of a wax gum base is disclosed in U.S. Patent No. 5,286,500.

In addition to a water insoluble gum base portion, a typical chewing gum composition includes a water soluble bulk portion and one or more flavouring agents. The water soluble portion can include bulk sweeteners, high intensity sweeteners, flavoring agents, softeners, emulsifiers, colors, acidulants, fillers, antioxidants, and other components that provide desired attributes.

Softeners are added to the chewing gum in order to optimize the chewability and mouthfeel of the gum. The softeners, which are also known as plasticizers and plasticizing agents, generally constitute between 0.5% to 15% by weight of the chewing gum. The softeners may include glycerin, lecithin, and combination thereof. Aqueous sweetener solutions such as those containing sorbitol, hydrogenated starch hydrolysates, com syrup and combinations thereof, may also be used as softeners and binding agents in chewing gum.

Bulk sweeteners include both sugar and sugarless components. Bulk sweeteners typically constitute 5% to 95% by weight of the chewing gum, more typically, 20% to 80% by weight, and more commonly, 30% to 60% by weight of the gum. Sugar sweeteners generally include saccharide components commonly known in the chewing gum art, including but not limited to, sucrose, dextrose, maltose, dextrin, dried invert sugar, fructose, levulose, galactose, com syrup solids, and the like, alone or in combination. Sugarless sweeteners include, but are not limited to, sugar alcohols such as sorbitol, mannitol, xylitol, hydrogenated starch hydrolysates, maltitol, and the like, alone or in combination.

High intensity artificial sweeteners can also be used, alone or in combination, with the above. Preferred sweeteners include, but are not limited to, sucralose, aspartame, NAPM derivatives such as neotame, salts of acesulfame, altitame, saccharin and its salts, cyclamic acid and its salts, glycyrrhizinate, dihydrochalcones, thaumatin, monellin, and the like, alone or in combination. In order to provide longer lasting sweetness and flavor perception, it may be desirable to encapsulate or otherwise control the release of at least a portion of the artificial sweetener. Such techniques as wet granulation, wax granulation, spray drying, spray chilling, fluid bed coating, coacervation, and fiber extension may be used to achieve the desired release characteristics.

Combinations of sugar and/or sugarless sweeteners may be used in chewing gum. Additionally, the softener may also provide additional sweetness such as with aqueous sugar or alditol solutions.

If a low calorie gum is desired, a low caloric bulking agent can be used. Examples of low caloric bulking agents include: polydextrose; Raftilose, Raftilin; Fructooligosaccharides (NutraFlora); Palatinose oligosaccharide; Guar Gum Hydrolysate (Sun Fiber); or indigestible dextrin (Fibersol). However, other low calorie bulking agents can be used.

A variety of flavoring agents can also be used, if desired. The flavor can be used in amounts of 0.1 to 15 weight percent of the gum, and preferably, 0.2% to 5% by weight Flavoring agents may include essential oils, synthetic flavors or mixtures thereof including, but not limited to, oils derived from plants and fruits such as citrus oils, fruit essences, peppermint oil, spearmint oil, other mint oils, dove oil, oil of wintergreen, anise and the like. Artificial flavouring agents and components may also be used. Natural and artificial flavoring agents may be combined in any sensorially acceptable fashion. Flavoring may include a cooling agent to enhance the flavor and perceived breath freshening of the product. Cooling agents include menthol, ethyl p-menthane carboxamide, N,2,3 - trimethyl-2-isopryl-butanamide, menthyl glutarate FEMA 4006, menthyl succinate, menthol PG carbonate, menthol EG carbonate, menthyl lactate, menthone glyceryl ketal, menthol glyceryl ether, N-tertbutyl-p-menthane-3-carboxamide, p-menthane-3-carboxylic acid glycerol ester, methyl-2-isopryl-bicyclo (2.2.1), heptane-2-carboxamide, menthol methyl ether and combinations thereof.

In addition, to the active ingredients of the present invention, additional active ingredients or medicaments may be added for various purposes. If the medicament or active is water soluble in the chewing gum, it preferably will include a base/emulsifier system which leads to the desired concentration of the medicament in the saliva (more hydrophilic balance). If the medicament or active is water insoluble, the chewing gum preferably includes a base/emulsifier system which leads to the desired concentration of the medicament in the saliva (more lipophilic balance).

In manufacturing the chewing gum including the active agent or ingredient, the active agent or medicament is added, preferably, early on in the mix. The smaller the amount of active ingredient used, the more necessary it becomes to preblend that particular ingredient to assume uniform distribution throughout the batch of gum. Whether a preblend is used or not, the active agent or medicament should be added within the first five minutes of mixing. For faster release, the active agent may be added late in the process.

Optionally, the chewing gum of the present invention may include additional breath freshening, anti-microbial or oral health ingredients. Food acceptable metallic salts selected from zinc and copper salts of gluconic acid, zinc and copper salts of lactic acid, zinc and copper salts of acetic acid, zinc and copper salts of citric acid and combinations thereof.

Anti-microbial essential oils and flavor components such as peppermint, methyl salicylate, thymol, eucalyptol, cinnamic aldehyde, polyphosphate, pyrophosphate and combinations thereof.

Dental health ingredients such as fluoride salts, phosphate salts, proteolytic enzymes, lipids, anti-microbials, calcium, electrolytes, protein additives, dental abrasives and combinations thereof.

In general, chewing gum is manufactured by sequentially adding the various chewing gum ingredients to a commercially available mixer known in the art. After the ingredients have been thoroughly mixed, the gum mass is discharged from the mixer and shaped into the desired form such as rolling sheets and cutting into sticks, extruding into chunks or casting into pellets, which are then coated or panned.

Generally, the ingredients are mixed by first melting the gum base and adding it to the running mixer. The base may also be melted in the mixer itself. Color or emulsifiers may also be added at this time. A softener such as glycerin may also be added at this time, along with syrup and a portion of the bulking agent. Further parts of the bulking agent are added to the mixer. Flavoring agents are typically added with the final portion of the bulking agent. Other optional ingredients are added to the batch in a typical fashion, well known to those of ordinary skill in the art.

The entire mixing procedure typically takes from five to fifteen minutes, but longer mixing times may sometimes be required. Those skilled in the art will recognize that many variations of the above described procedure may be followed.

Chewing gum base and chewing gum product have been manufactured conventionally using separate mixers, different mixing technologies and, often, at different factories. One reason for this is that the optimum conditions for manufacturing gum base, and for manufacturing chewing gum from gum base and other ingredients such as sweeteners and flavors, are so different that it has been impractical to integrate both tasks. Chewing gum base manufacture, on the one hand, involves the dispersive (often high shear) mixing of difficult-to-blend ingredients such as elastomer, filler, elastomer plasticizer, base softeners/emulsifiers and sometimes wax, and typically requires long mixing times. Chewing gum product manufacture, on the other hand, involves combining the gum base with more delicate ingredients such as product softeners, bulk sweeteners, high intensity sweeteners and flavoring agents using distributive (generally lower shear) mixing, for shorter periods.

The following are examples of formulations of cardamom oil in chewing gum. The examples are not intended to exclude other variations in formulations and the present invention is not limited to these formulations.

**Table 1. Antimicrobial Gum Formulas (% by weight)**

| Ingredient | Ex 1 | Ex 2 | Ex 3 | Ex 4 | Ex 5 |
|---|---|---|---|---|---|
| Gum Base | 26.00 | 26.00 | 26.00 | 27.5 | 27.5 |
| Talc powder | 3.00 | 3.00 | 3.00 | 3.00 | 3.00 |
| Glycerine | 0.50 | 0.50 | 0.50 | 0.50 | 0.50 |
| Sorbitol | 49.71 | 50.96 | 48.71 | 48.71 | 44.71 |
| Glycerol | 15.01 | 15.01 | 15.01 | 15.01 | 15.01 |
| Mannitol | 1.52 | 1.52 | 1.52 | 1.52 | 1.52 |
| Maltitol | 0.76 | 0.76 | 0.76 | 0.76 | 0.76 |
| Water | 1.18 | 1.18 | 1.18 | 1.18 | 1.18 |
| Aspartarme | 0.53 | 0.53 | 0.53 | 0.53 | 0.53 |
| Color | 0.01 | 0.01 | 0.01 | 0.01 | 0.01 |
| Zein | 0.04 | 0.04 | 0.04 | 0.04 | 0.04 |
| NaOH | 0.01 | 0.01 | 0.01 | 0.01 | 0.01 |
| Acesul-phame Potassium | 0.13 | 0.13 | 0.13 | 0.13 | 0.13 |
| Cardamom Oil | 1.50 | 0.25 | 2.50 | 1.00 | 5.00 |
| Hydroxy-propylmethyl-cellulose | 0.10 | 0.10 | 0.10 | 0.10 | 0.10 |
| | | | | | |
| Total % | 100.00 | 100.00 | 100.00 | 100.00 | 100.00 |

**Table 2. Antimicrobial Gum Formulas (% by weight)**

| Ingredient | Ex 6 | Ex 7 | Ex 8 | Ex 9 | Ex 10 |
|---|---|---|---|---|---|
| Gum Base | 19.46 | 20.71 | 19.46 | 19.46 | 18.46 |
| Sugar | 62.13 | 62.13 | 61.13 | 62.63 | 61.63 |
| Com Syrup | 15.57 | 15.57 | 15.57 | 15.57 | 13.57 |
| Color | 0.67 | 0.67 | 0.67 | 0.67 | 0.67 |
| P.A. | 0.67 | 0.67 | 0.67 | 0.67 | 0.67 |
| Cardamom Oil | 1.50 | 0.25 | 2.50 | 1.00 | 5.00 |
| | | | | | |
| Total % | 100.00 | 100.00 | 100.00 | 100.00 | 100.00 |

In yet another embodiment of the present invention, and effective amount for anti-microbial benefit of cardamom oil is present in a confectionery formulation. The cardamom oil is present in an amount of from 0.01% to 3%. by weight of the confectionery product. In a preferred embodiment of the present invention, the amount of cardamom oil is about 1% of the weight of the confectionery product. In another preferred embodiment, the cardamom oil is present in the amount of about 0.01% by weight of the confectionery product.

Confectionery products for this invention may be hard candies, chewy candies, coated chewy center candies and tabletted candies. By way of example, the hard candy is primarily comprised of corn syrup and sugar, and derives its name from the fact that it contains only 1.0% and 4% moisture. In appearance, these types of candies are solid, but they are actually supercooled liquids, which are far below their melting points. There are different types of hard candies. Glass types are usually dear or made opaque with dyes; and Grained Types, which are always opaque.

The continuous making process of the Deposited Glass Types, with a sugar base are as follows. Sugar com syrup mixture is spread over a cylinder heated by high pressure steam. Rapid head exchange causes the water in the syrup to evaporate. The cooked syrup is discharged, colors and flavors are added. These can be conveyed directly to hoppers which then discharge directly into molds.

The candy is conveyed to batch rollers, which shapes and sizes the batch. The candy enters a former, which shapes the individual pieces into discs, balls, barrels, etc. The present invention can be made into any shape, circles, squares, triangles etc, also into animal shapes or any other novelty molding available. The candy is then cooled, wrapped and packaged.

For Grained Types of candy, water and sugar are the basic components being mixed with other ingredients, and cooked at high temperatures (143°C, 154°C) (290°F 310°F) causing the water to turn to steam. The product is transferred to a cooling wheel, where it is collected in about 68kg (150 pound) batches, placed in a pulling machine to aerate the product, and the flavor is added. The candy is transferred to batch rollers where it is shaped and sized. The candy then enters a former, which shapes the individual pieces. The candy is cooled at a relative humidity of 35% and enters a rotating drum where it is coated with a fine sugar. The candy is then conveyed to the graining room for four hours at 32°C (90°F) and 60% humidity. The entrapped air and moisture causes the product to grain.

The present invention can be of a variety of shapes, flavors and sizes. The present invention may contain sugar or may be sugarless.

Flavors used in the present invention may be peppermint oils, citrus oils, arvensis, fruit flavors, spearmint oils and the like.

Colors used in the present invention are colorants are typically known as FD&C dyes and lakes.

By way of example and not limitation, the following examples illustrate various embodiments of the confectionery formulations of the present invention.

**Table 6. Antimicrobial Candy Formulations (% by weight)**

| Ingredient | Ex 26 | Ex 27 | Ex 28 | Ex 29 | Ex 30 |
|---|---|---|---|---|---|
| Corn Syrup | 44.51 | 43.25 | - | - | 48.00 |
| Sugar | 53.49 | 50.00 | - | - | 47.00 |
| Polyalcohols | - | - | 95.20 | 95.77 | - |
| Flavor | 1.00 | 5.00 | 3.00 | 2.00 | 2.50 |
| Color | 0.50 | 1.00 | 0.60 | 0.80 | 0.50 |
| Cardamom oil | 0.50 | 0.75 | 1.00 | 1.23 | 2.00 |
| High Intensity Sweetener | - | - | 0.20 | 0.20 | - |
| | | | | | |
| Total % | 100.00 | 100.00 | 100.00 | 100.00 | 100.00 |

## Claims

1. A Chewing gum composition comprising:
a) a gum base ;
b) a flavour ;
c) a sweetener ; and
d) cardamom oil present in an amount of from 0.01 to 5% by weight of the chewing gum composition.

2. The chewing gum composition of claim 1, wherein the - cardamom oil is present in an amount of from 0.01 to 1.0% by weight of the chewing gum composition.

3. The chewing gum composition of claim 1, wherein the amount of cardamom oil is present in an amount of from 0.01 to 0.25% by weight of the chewing gum composition.

4. The chewing gum composition of any one of claims 1 to 3, further comprising a food acceptable zinc and copper salt of acids selected from the group consisting of gluconic acid, lactic acid, acetic acid, citric acid and combinations thereof.

5. The chewing gum composition of any one of claims 1 to 4, further comprising pyrophosphate or polyphosphate.

6. The chewing gum composition of any one of claims 1 to 5 wherein said cardamom oil is encapsulated.

7. The chewing gum composition of any one of claims 1 to 5, wherein said cardamom oil is spray dried.

8. The chewing gum composition of any one of claims 1 to 5, wherein said chewing gum composition is coated.

9. The chewing gum composition of claim 8, wherein said cardamom oil it present in said coating.

10. The chewing gum composition of claim 8, wherein said cardamom oil is encapsulated.

11. The chewing gum composition of any one of claims 1 to 10, further comprising a high intensity sweetener selected from the group consisting of sucralose, aspartame, NAPM derivatives such as neotame, salts of acesulfame, alitam saccharin and its salts, cyclamic acid and its salts, glycyrrhizinate, dihydrochalcones, thaumatin, monellin, and combinations thereof,

12. The chewing gum composition of any one of claims 1 to 11, further comprising a medicament.

13. The chewing gum composition of any one of claims 1 to 12, further comprising an active agent.

14. The chewing gum composition of any one of claims 1 to 13, further comprising a cooling agent selected from the group consisting of menthol, ethyl p-menthane carboxamide, N,2,3-trimethyl-2-isopryl-butanamide, menthyl glutarate FEMA 4006, menthyl succinate, menthol PG carbonate, menthol EG carbonate, menthyl lactate, menthone glyceryl ketal, menthol glyceryl ether, N-tertbutyl-p-menthane-3-carboxamide, p-menthane-3-carboxylic acid glycerol ester, methyl-2-isopryl-bicyclo (2.2.1), heptane-2-carboxamide, menthol methyl ether and combinations thereof.

15. The chewing gum composition of any one of claims 1 to 14, formulated to deliver at least 0.005% concentration of cardamom oil to the oral cavity.

16. The chewing gum composition of any one of claims 1 to 15, further comprising an oral health ingredient.

17. A confectionery composition, comprising cardamom oil, wherein said cardamom oil is present in an amount of from 0.01 to 3% by weight of the confectionary composition.

18. The confectionery composition of claim 17, wherein said cardamom oil is present in an amount of from 0.01 to 1% by weight of the confectionary composition.

19. The confectionery composition of claims 17 or 18, wherein the form of said confectionery composition is a hard candy.

20. The confectionery composition of claim 19, wherein said hard candy contains 1.0% to 4% moisture by weight of the confectionery composition.

21. The confectionery composition of claims 17 or 18, wherein the form of said confectionery composition is a chewing candy.

22. The confectionery composition of claims 17 or 18, wherein the form of said confectionery composition is a coated chewy center candy.

23. The confectionery composition of claims 17 or 18, wherein the form of said confectionery composition is a tabletted candy.

24. The confectionery composition of any one of claims 17 to 23, further comprising a color.

25. The confectionery composition of any one of claims 17 to 24, further comprising a flavour.

26. A composition as defined in any one of claims 1 to 25 for use in a method of oral cleansing of the oral cavity.

27. Use of a composition as defined in any one of claims 1 to 25 for the manufacture of a medicament for oral cleansing of the oral cavity.

## Patentansprüche

1. Kaugummi-Zusammensetzung, welche umfasst:
a) eine Gummibasis;
b) ein Aroma;
c) ein Süßungsmittel; und
d) Kardamomöl, welches in einer Menge von 0,01 bis 5 Gewichts-% der Kaugummi-Zusammensetzung vorhanden ist.

2. Kaugummi-Zusammensetzung nach Anspruch 1, wobei Kardamomöl in einer Menge von 0,01 bis 1,0 Gewichts-% der Kaugummi-Zusammensetzung vorhanden ist.

3. Kaugummi-Zusammensetzung nach Anspruch 1, wobei die Menge an Kardamomöl in einer Menge von 0,01 bis 0,25 Gewichts-% der Kaugummi-Zusammensetzung vorhanden ist.

4. Kaugummi-Zusammensetzung nach einem der Ansprüche 1 bis 3, welche weiterhin ein für Lebensmittel zulässiges Zink- und Kupfersalz von Säuren umfasst, die aus der Gruppe bestehend aus Gluconsäure, Milchsäure, Essigsäure, Zitronensäure und Kombinationen davon ausgewählt sind.

5. Kaugummi-Zusammensetzung nach einem der Ansprüche 1 bis 4, welche weiterhin Pyrophosphat oder Polyphosphat umfasst.

6. Kaugummi-Zusammensetzung nach einem der Ansprüche 1 bis 5, wobei das Kardamomöl verkapselt ist.

7. Kaugummi-Zusammensetzung nach einem der Ansprüche 1 bis 5, wobei das Kardamomöl sprühgetrocknet ist.

8. Kaugummi-Zusammensetzung nach einem der Ansprüche 1 bis 5, wobei die Kaugummi-Zusammensetzung beschichtet ist.

9. Kaugummi-Zusammensetzung nach Anspruch 8, wobei das Kardamomöl in der Beschichtung vorhanden ist.

10. Kaugummi-Zusammensetzung nach Anspruch 8, wobei das Kardamomöl verkapselt ist.

11. Kaugummi-Zusammensetzung nach einem der Ansprüche 1 bis 10, welche weiterhin ein Süßungsmittel mit hoher Intensität umfasst, welches aus der Gruppe bestehend aus Sucralose, Aspartam, NAPM-Derivaten, wie z. B. Neotam, Salzen von Acesulfam, Alitam, Saccharin und seinen Salzen, Cyclamsäure und seinen Salzen, Glycyrrhizinat, Dihydrochalconen, Thaumatin, Monellin und Kombinationen davon, ausgewählt ist.

12. Kaugummi-Zusammensetzung nach einem der Ansprüche 1 bis 11, welche weiterhin ein Medikament umfasst.

13. Kaugummi-Zusammensetzung nach einem der Ansprüche 1 bis 12, welche weiterhin einen Wirkstoff umfasst.

14. Kaugummi-Zusammensetzung nach einem der Ansprüche 1 bis 13, welche weiterhin ein Kühlungsmittel umfasst, welches aus der Gruppe bestehend aus Menthol, Ethyl-p-menthan-carboxamid, N,2,3-Trimethyl-2-isopropyl-butanamid, Menthylglutarat FEMA 4006, Menthylsuccinat, Menthol-PG-carbonat, Menthol-EG-carbonat, Menthyllactat, Menthon-glyceryl-ketal, Menthol-glyceryl-ether, N-tert-Butyl-p-menthan-3-carboxamid, p-Menthan-3-carbonsäure-glycerin-ester, Methyl-2-isopropyl-bicyclo(2.2.1), Heptan-2-carboxamid, Mentholmethyl-ether und Kombinationen davon, ausgewählt ist.

15. Kaugummi-Zusammensetzung nach einem der Ansprüche 1 bis 14, welche so formuliert ist, dass mindestens 0,005% der Konzentration an Kardamomöl der Mundhöhle zugeführt wird.

16. Kaugummi-Zusammensetzung nach einem der Ansprüche 1 bis 15, welche weiterhin einen Bestandteil für die orale Gesundheit umfasst.

17. Süßwaren-Zusammensetzung, welche Kardamomöl umfasst, wobei das Kardamomöl in einer Menge von 0,01 bis 3 Gewichts-% der Süßwaren-Zusammensetzung vorhanden ist.

18. Süßwaren-Zusammensetzung nach Anspruch 17, wobei das Kardamomöl in einer Menge von 0,01 bis 1 Gewichts-% der Süßwaren-Zusammensetzung vorhanden ist.

19. Süßwaren-Zusammensetzung nach den Ansprüchen 17 oder 18, wobei die Form der Süßwaren-Zusammensetzung ein Bonbon ist.

20. Süßwaren-Zusammensetzung nach Anspruch 19, wobei das Bonbon 1,0% bis 4% an Feuchtigkeit bezogen auf das Gewicht der Süßwaren-Zusammensetzung enthält.

21. Süßwaren-Zusammensetzung nach den Ansprüchen 17 oder 18, wobei die Form der Süßwaren-Zusammensetzung ein Kaubonbon ist.

22. Süßwaren-Zusammensetzung nach den Ansprüchen 17 oder 18, wobei die Form der Süßwaren-Zusammensetzung ein beschichtetes Bonbon mit einem kaubaren Zentrum ist.

23. Süßwaren-Zusammensetzung nach den Ansprüchen 17 oder 18, wobei die Form der Süßwaren-Zusammensetzung ein tablettiertes Bonbon ist.

24. Süßwaren-Zusammensetzung nach einem der Ansprüche 17 bis 23, welche weiterhin eine Farbe umfasst.

25. Süßwaren-Zusammensetzung nach einem der Ansprüche 17 bis 24, welche weiterhin ein Aroma umfasst.

26. Zusammensetzung, wie in einem der Ansprüche 1 bis 25 definiert, zur Verwendung in einem Verfahren der oralen Reinigung der Mundhöhle.

27. Verwendung einer Zusammensetzung, wie in einem der Ansprüche 1 bis 25 definiert, zur Herstellung eines Medikaments zur oralen Reinigung der Mundhöhle.

## Revendications

1. Composition de gomme à mâcher comprenant :
a) une gomme base ;
b) un aromatisant ;
c) un édulcorant ; et
d) de l'essence de cardamome présente en une quantité de 0,01 à 5 % en poids de la composition de gomme à mâcher.

2. Composition de gomme à mâcher selon la revendication 1, dans laquelle l'essence de cardamome est présente en une quantité de 0,01 à 1,0 % en poids de la composition de gomme à mâcher.

3. Composition de gomme à mâcher selon la revendication 1, dans laquelle la quantité d'essence de cardamome est présente en une quantité de 0,01 à 0,25 % en poids de la composition de gomme à mâcher.

4. Composition de gomme à mâcher selon l'une quelconque des revendications 1 à 3, comprenant de plus un sel de zinc et de cuivre acceptable pour l'usage alimentaire d'acides choisis dans le groupe formé par l'acide gluconique, l'acide lactique, l'acide acétique, l'acide citrique et des associations d'entre eux.

5. Composition de gomme à mâcher selon l'une quelconque des revendications 1 à 4, comprenant de plus un pyrophosphate ou un polyphosphate.

6. Composition de gomme à mâcher selon l'une quelconque des revendications 1 à 5, dans laquelle ladite essence de cardamome est encapsulée.

7. Composition de gomme à mâcher selon l'une quelconque des revendications 1 à 5, dans laquelle ladite essence de cardamome est séchée par atomisation.

8. Composition de gomme à mâcher selon l'une quelconque des revendications 1 à 5, dans laquelle ladite composition de gomme à mâcher est enrobée.

9. Composition de gomme à mâcher selon la revendication 8, dans laquelle ladite essence de cardamome est présente dans ledit enrobage.

10. Composition de gomme à mâcher selon la revendication 8, dans laquelle ladite essence de cardamome est encapsulée.

11. Composition de gomme à mâcher selon l'une quelconque des revendications 1 à 10, comprenant de plus un édulcorant à haute intensité choisi dans le groupe formé par le sucralose, l'aspartame, les dérivés de NAPM tels que le néotame, les sels d'acésulfame, l'alitame, la saccharine et ses sels, l'acide cyclamique et ses sels, le glycyrrhizinate, les dihydrochalcones, la thaumatine, la monelline et leurs associations.

12. Composition de gomme à mâcher selon l'une quelconque des revendications 1 à 11, comprenant de plus un médicament.

13. Composition de gomme à mâcher selon l'une quelconque des revendications 1 à 12, comprenant de plus un agent actif.

14. Composition de gomme à mâcher selon l'une quelconque des revendications 1 à 13, comprenant de plus un agent rafraîchissant choisi dans le groupe formé par le menthol, l'éthyl-p-menthane-carboxamide, le N-2,3-triméthyl-2-isopropyl-butanamide, le glutarate de menthyle FEMA 4006, le succinate de menthyle, le carbonate de menthol et de PG, le carbonate de menthol et d'EG, le lactate de menthyle, le cétal glycérylique de menthone, l'éther glycérylique de menthol, le N-*tert*-butyl-*p*-menthane-3-carboxamide, l'ester de glycérol d'acide *p*-menthane-3-carboxylique, le méthyl-2-isopropyl-bicyclo(2.2.1)heptane-2-carboxamide, l'éther méthylique de menthol et leurs associations.

15. Composition de gomme à mâcher selon l'une quelconque des revendications 1 à 14, formulée pour délivrer une concentration d'au moins 0,005 % d'essence de cardamome à la cavité buccale.

16. Composition de gomme à mâcher selon l'une quelconque des revendications 1 à 15, comprenant de plus un ingrédient d'hygiène buccale.

17. Composition de confiserie comprenant de l'essence de cardamome, dans laquelle ladite essence de cardamome est présente en une quantité de 0,01 à 3 % en poids de la composition de confiserie.

18. Composition de confiserie selon la revendication 17, dans laquelle ladite essence de cardamome est présente en une quantité de 0,01 à 1 % en poids de la composition de confiserie.

19. Composition de confiserie selon la revendication 17 ou 18, dans laquelle la forme de ladite composition de confiserie est un bonbon dur.

20. Composition de confiserie selon la revendication 19, dans laquelle ledit bonbon dur contient 1,0 % à 4 % en poids d'humidité par rapport au poids de la composition de confiserie.

21. Composition de confiserie selon la revendication 17 ou 18, dans laquelle la forme de ladite composition de confiserie est un bonbon à mâcher.

22. Composition de confiserie selon la revendication 17 ou 18, dans laquelle la forme de ladite composition de confiserie est un bonbon enrobé ayant un noyau à mâcher.

23. Composition de confiserie selon la revendication 17 ou 18, dans laquelle la forme de ladite composition de confiserie est un bonbon pastillé.

24. Composition de confiserie selon l'une quelconque des revendications 17 à 23, comprenant de plus un colorant.

25. Composition de confiserie selon l'une quelconque des revendications 17 à 24, comprenant de plus un aromatisant.

26. Composition telle que définie dans l'une quelconque des revendications 1 à 25, pour son utilisation dans une méthode de nettoyage buccal de la cavité buccale.

27. Utilisation d'une composition telle que définie dans l'une quelconque des revendications 1 à 25, pour la fabrication d'un médicament destiné au nettoyage buccal de la cavité buccale.
